(19) Europäisches Patentamt
European Patent Office
Office européen des brevets

(11) Veröffentlichungsnummer: **0 007 591**
**B1**

(12) # EUROPÄISCHE PATENTSCHRIFT

(45) Veröffentlichungstag der Patentschrift: **28.07.82**

(51) Int. Cl.³: **A 61 K 31/635** // (A61K31/635, 31/505)

(21) Anmeldenummer: **79102561.2**

(22) Anmeldetag: **20.07.79**

(54) **Stabile flüssige Arzneimittelformulierung auf Basis von 2,6-Diamino-5-(3',4',5'-trimethoxybenzyl)-pyrimidin(Trimethoprim) und einem Sulfonamid, Verfahren zu ihrer Herstellung und ihre Verwendung.**

(30) Priorität: **01.08.78 CH 8216/78**

(43) Veröffentlichungstag der Anmeldung: **06.02.80 Patentblatt 80/3**

(45) Bekanntmachung des Hinweises auf die Patenterteilung: **28.07.82 Patentblatt 82/30**

(84) Benannte Vertragsstaaten: **BE CH DE FR GB IT LU NL**

(56) Entgegenhaltungen:
**keine**
**DE-A-2 704 708**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Patentabteilung Postfach, CH-4002 Basel (CH)**

(72) Erfinder: **Lukas, Gerhard, Dr., In den Wegscheiden 16, CH-4132 Muttenz (CH)**
Erfinder: **Atasoy, Kaya, Dr., Loogstrasse 32, CH-4142 Münchenstein (CH)**

**EP 0 007 591 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Stabile flüssige Arzneimittelformulierung auf Basis von 2,6-Diamino-5-(3',4',5'-trimethoxybenzyl)-pyrimidin (Trimethoprim) und einem Sulfonamid, Verfahren zu ihrer Herstellung und ihre Verwendung

Die vorliegende Erfindung betrifft eine flüssige Formulierung, enthaltend eine Wirkstoffkombination aus 2,6-Diamino-5-(3',4',5'-trimethoxybenzyl)-pyrimidin (Trimethoprim) und einem Sulfon-amid zur Bekämpfung von bakteriellen und protozoären Krankheiten bei Tieren mittels Trinkwasserapplikation. Ausser den genannten Wirkstoffen enthält die Formulierung ferner ein organisches Lösungsmittel, einen Lösungsvermittler, ein Tensid und Wasser.

Die Wirksamkeit von Sulfonamid-Trimethoprim-Kompositionen in der Bekämpfung von Infektionskrankheiten bei Mensch und Tier ist gut bekannt. Die besondere Bedeutung der Trimethoprim-Komponente beruht auf der Potenzierung der antibakteriellen bzw. protozoären Wirksamkeit der Sulfonamide.

Die Verabreichung solcher Wirkstoffkombinationen an Tiere stösst indessen auf mancherlei Schwierigkeiten. So ist bei der Applikation in fester Form durch Zugabe zur Alimentation in den überwiegenden Fällen eine erfolgversprechende Therapie nicht möglich, da erkrankte Tiere im allgemeinen die Futteraufnahme stark reduzieren, wenn nicht gar gänzlich verweigern. Die Wasseraufnahme erleidet dagegen normalerweise keine Einschränkung, so dass sich durch Medikierung des Trinkwassers ein Weg für die enterale Einschleusung von Wirkstoffen in den Tierorganismus anbietet.

Eine weitere Möglichkeit ist die parenterale Wirkstoffapplikation mittels Injektion, die jedoch bei prophylaktischer Anwendung auf zahlenmässig umfangreiche Tiergruppen, die in der modernen Nutztierhaltung im Vordergrund steht, aus Kostengründen nicht praktikabel ist. Auch hier stellt die Trinkwassermedikierung eine sinnvolle, nützliche Alternative dar. Es zeigt sich also, dass der Bereitstellung einer den praktischen Bedürfnissen angepassten stabilen Trinkwasserlösung von Sulfonamid-Trimethoprim-Kombinationen für die Bekämpfung von Infektionserkrankungen bei Tieren eine wichtige Bedeutung zukommt. Darüber hinaus stellt die Trinkwassermedikierung für einige Nutztierarten wie beispielsweise Geflügel in der Praxis die einzige Möglichkeit zur Therapie grösserer Tiergruppen dar.

Die Bereitstellung von therapeutisch brauchbaren Trinkwasser-Lösungen eines Sulfonamids in Kombination mit Trimethoprim bereitet indessen beträchtliche Schwierigkeiten, wobei sowohl Mängel in der Beständigkeit der Konzentrate als auch die Instabilität vor allem der verteilungskonform verdünnten Wirkstofflösungen eine Hauptrolle spielen. Das gilt umso mehr als heutzutage in immer stärkerem Masse automatische Tränkeeinrichtungen Eingang in die Praxis finden. Ein reibungsloses Funktionieren derartiger Vorrichtungen hängt jedoch u.a. auch von der einwandfreien Beschaffenheit der zur Verteilung gelangenden Lösungen ab. So führt das Auftreten von Ausfällungen wie festen Niederschlägen in den für die Herstellung der Endverdünnung vorgesehenen Lösungen zwangsläufig zu Schwankungen in der Dosierung, wodurch eine zuverlässige kontrollierte Therapie nicht mehr gewährleistet ist. Ferner kann das Auftreten von festen Bestandteilen in den Zwischenverdünnungen der die Wirkstoffe enthaltenden Trinkwasserlösungen Störungen in den Verteilungsapparaturen zur Folge haben, was mit Verlusten an wertvollem Material und zusätzlichem Arbeitsaufwand verbunden ist.

Da nun weder Trimethoprim noch die Sulfonamide eine ausreichende Wasserlöslichkeit besitzen, werden im allgemeinen die gut wasserlöslichen Salze dieser Verbindungen verwendet. Hierbei ergibt sich jedoch das Problem, dass bei Kombination der gebildeten Salzlösungen durch Annäherung des pH-Wertes an den Neutralpunkt eine Ausfällung der Wirksubstanzen stattfindet. Zur Behebung dieser Schwierigkeiten ist bereits mehrfach vorgeschlagen worden, Wirkstoffkombinationen bestehend aus Trimethoprim und jeweils verschiedenen Sulfonamiden unter Verwendung von organischen Lösungsmitteln bzw. Lösungsvermittlern in eine für therapeutische Zwekke geeignete Form zu bringen. So wird in der deutschen Offenlegungsschrift Nr. 2 445 401 die Herstellung von für Injektionszwecke bestimmten wasserfreien Wirkstoffkombinationslösungen mit einem Gemisch von Sulfadimidin und Sulfathiazol als Sulfonamidkomponente mit einer Konzentration von 24–48% beschrieben.

Die deutsche Offenlegungsschrift Nr. 2 311 214 führt neben der Bildung von festen Sulfonamid-Trimethoprim-Kombinationen als Futterzusatz die Herstellung einer wasserarmen Injektionslösung mit einem Gesamtwirkstoffgehalt von ca. 25% an, wobei N-(4,5-Dimethyl-3-isoxazolyl)-sulfanilamid als Sulfonamid fungiert. Erwähnt werden dabei auch wässrige Formulierungen, bei denen der Gehalt der genannten Wirkstoffkombination für orale Anwendung durch Verdünnen mit Wasser auf 5% und darunter gesenkt werden kann.

Die britische Patentschrift Nr. 1 176 395 berichtet über injizierbare Formulierungen von Trimethoprim mit einer Reihe von Sulfonamiden wie 5,6-Dimethoxy-4-sulfanilamidpyrimidin, 4,6-Dimethyl-2-sulfanilamidpyrimidin, 2,4-Dimethoxy-6-sulfanilamidpyrimidin oder 3,4-Dimethyl-5-sulfonamidisoxazol mit Wirkstoffkonzentrationen von 12–14%. Für die Herstellung dieser Formulierungen müssen zuerst die Wirkstoffkomponenten voneinander getrennt in die Lösung gebracht werden, wobei die Sulfonamide entweder zusammen mit Basen oder in einem Fall als Na-Salz in Wasser und das Trimethoprim in organischen Lösungsmitteln oder Lösungsvermittlern gelöst werden, um dann miteinander vermischt zu werden.

Dagegen wird für die Herstellung der in der deutschen Offenlegungsschrift Nr. 2 400 218 beschriebenen flüssigen Formulierungen von Trimethoprim mit Sulfamethazol oder Sulfacetamid eine andere Verfahrensweise angegeben. So wird

zuerst das Trimethoprim unter Zusatz von Säure in Wasser gelöst und dann das Sulfonamid zusammen mit organischen Lösungsmitteln bzw. Lösungsvermittlern hinzugegeben. Die so hergestellten Wirkstoffkombinationen werden gemäss der obengenannten Patentschrift zur Injektion und für orale Applikation vorgeschlagen. Ihre Wirkstoffkonzentration beträgt ca. 8–10% und zum Teil auch weniger als 1%.

Eine Überprüfung der in der deutschen Offenlegungsschrift Nr. 2 311 214 und der britischen Patentschrift Nr. 1 176 395 vorgeschlagenen wasserhaltigen Formulierungen konnte jedoch hinsichtlich ihrer Träger- und Verteilungsstoffe bei Anwendung auf die Wirkstoffkombinationen der vorliegenden Erfindung nicht befriedigen, so dass in der Praxis brauchbare stabile und klare wässrige Lösungen mit diesen Wirkstoffen nicht herstellbar waren. Desgleichen haben sich die in der deutschen Offenlegungsschrift Nr. 2 704 708 beschriebenen wasserhaltigen Sulfonamid/Patentierformulierungen bei Überprüfung in Verdünnungen mit Trinkwasser auf die gemäss der vorliegenden Erfindung vorgeschriebenen und den Bedürfnissen der Praxis entsprechenden Konzentrationen als nicht stabil erwiesen.

Die Vorbeschreibungen betreffen also vor allem Sulfonamid-Trimethoprim-Formulierungen höherer Konzentration mit und ohne Wasserzusatz, die in erster Linie für die Injektionsanwendung vorgesehen sind, jedoch mitunter auch für die Trinkwasserapplikation empfohlen werden. In den meisten Fällen ist dafür die Verdünnung mit Wasser notwendig, um den Wirkstoffgehalt auf die für diese Verwendung erforderliche geringere Konzentration zu bringen, wobei es im Konzentrationsbereich der für automatische Tränkevorrichtungen notwendigen Zwischenverdünnungen mehr oder weniger schnell zu Ausfällungen kommt, was die Unbrauchbarkeit dieser Lösungen zur Folge hat. Bei den bereits für die Trinkwasserapplikation vorgeschlagenen Formulierungen besteht der Nachteil, dass die Zwischenverdünnungen nur sehr kurze Zeit klar bleiben und keine Aufbewahrung gestatten, was je nach Applikationsfolge somit die mehr oder weniger häufige Herstellung von frischen, zum möglichst raschen Verbrauch bestimmten Wirkstofflösungen in Wasser unabdingbar macht.

Das führt in der Praxis vor allem bei gleichzeitiger Behandlung grösserer Gruppen von erkrankten Tieren sowie bei prophylaktischer Medikierung, die im allgemeinen auf breiter Basis durchgeführt wird, zu einem sehr nachteiligen arbeits- und kostenträchtigen Aufwand.

Ausserdem wird in Publikationen darauf hingewiesen, dass einige der in den vorgenannten Patentschriften bzw. Offenlegungsschriften als funktionelle Hilfsstoffe vorgeschlagenen Lösungsmittel bzw. Lösungsvermittler wie beispielsweise N,N-Dimethylacetamid, Propylenglykol, Dimethylformamid, Glycerinformal und Polyäthylenglykole aus pharmakologisch-toxischer Sicht als nicht ganz unbedenklich angesehen werden können [siehe dazu DE-OS 2 631 779; H.P. Fiedler, Lexikon der Hilfsstoffe für Pharmazie, Kosmetik, 1971; Prax. Pneumolog. 28 (1971) 491].

Ausgehend von den bereits erwähnten Vorteilen, die in der Verabreichung von Medikamenten von Sulfonamid-Trimethoprim-Kombinationen zur prophylaktischen und therapeutischen Behandlung hinsichtlich der Trinkwasserapplikation gegenüber der Verabreichung in Form von Zusätzen zu Trockenfutter oder Injektionen a priori bestehen, ergibt sich das dringende Bedürfnis wässrige Lösungen der genannten Wirkstoffkombinationen herzustellen, die bezüglich Klarheit und Löslichkeit in einem breiten Konzentrationsbereich und über einen längeren Zeitraum stabil sind, um den Erfordernissen der Praxis besser als bisher zu genügen.

Zur Lösung dieser Aufgabe werden deshalb mit Wasser verdünnbare flüssige Formulierungen folgender Zusammensetzung vorgeschlagen.

2,6-Diamino-5-(3′,4′,5′-trimethoxybenzyl)-pyrimidin (Trimethoprim) und ein Sulfonamid der Formel I

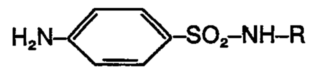

(I)

in welcher

R 6-Chlor-2-pyrazinyl oder 6-Chlor-3-pyridazinyl bedeutet, oder ein Salz davon im Mengenverhältnis 1:1 bis 1:20, vorzugsweise 1:4 bis 1:5, mit einem Gesamtanteil von 15 bis 25 Gew.% gelöst in einem physiologisch verträglichen, mit Wasser mischbaren Lösungsmittel, einem Lösungsvermittler, einem Tensid und Wasser und für den Fall, dass das Sulfonamid nicht als Salz eingesetzt wird, einer Base, dadurch gekennzeichnet, dass als Lösungsmittel N-Methylpyrrolidon allein oder in Kombination mit Glykolmonoäthyläther neben Wasser, als Tensid Bis-(2-äthylhexyl)-Na-sulfosuccinat und als Lösungsvermittler einen aus der Gruppe Hydroxyäthyltheophyllin, Nicotinsäureamid oder Na-Benzoat und als Base Äthanolamin verwendet werden, wobei die verwendete Menge des Lösungsmittels 60–80 Gew.-%, des Tensids 0,1–1,0 Gew.-% und des Lösungsvermittlers 1–10 Gew.-% bezogen auf das Gesamtgewicht der Formulierung beträgt. Bei den zur Anwendung gelangenden Lösungsvermittlern sind folgende Mengen als bevorzugt anzusehen für Hydroxyäthyltheophyllin und Na-Benzoat 1–10 Gew.-% und Nicotinsäureamid 2–4 Gew.-%.

Unter den beiden vorstehend genannten Wirkstoffkombinationen ist diejenige neu, die neben Trimethoprim als Sulfonamid N-[6-Chlorpyrazinyl-(2)]-sulfanilamid (Sulfachlorpyrazin) enthält, während eine Kombination enthaltend ausser Trimethoprim als Sulfonamid N-[6-Chlorpyridazinyl-(3)]-sulfanilamid (Sulfachlorpyridazin) bezüglich ihrer Wirkstoffe bereits vorbeschrieben ist [vgl. Magyar Allatorvosok Lapja 12 (1975) 833–836]. In der erfindungsgemäss vorgeschlagenen speziellen Komposition zusammen mit den verwendeten funktionellen Hilfsstoffen ist sie jedoch neu.

Mit den vorliegenden erfindungsgemässen Formulierungen ist es gelungen, konzentrierte wasserhaltige Lösungen herzustellen, die nicht nur über längere Zeit klar bleiben und unter normalen

Bedingungen unbeschadet lagerungsfähig sind, sondern auch noch nach Verdünnung mit Wasser über einen breiten Konzentrationsbereich hinweg überraschenderweise ihre Stabilitätseigenschaften bewahren. So sind einerseits die konzentrierten Formen den lagerungs- und transportbedingten Belastungen gegenüber stabil, während anderseits die verdünnten Lösungen als Zwischenverdünnungen über eine dem Bedarf angepasste Zeitspanne von mehreren Tagen am Einsatzort stabil aufbewahrt werden können, um dann ohne besonderen Aufwand in die gewünschte, den therapeutischen Erfordernissen entsprechende Endverdünnung überführt zu werden. Der dadurch ermöglichte einfache und ohne Verluste an wertvollen Wirkstoffen arbeitende Dosierungs- und Applikationsmodus dient insbesondere einer Verbesserung der therapeutischen Effizienz durch präzisere Wirkstoffdosierung sowie der Verminderung des Aufwands an Material und Arbeit.

Das auf den erfindungsgemässen Formulierungen basierende vorteilhafte Verdünnungsschema wird durch folgende Verhältnisse charakterisiert: In den Formulierungen beträgt der Anteil an Gesamtwirkstoff 15–25 Gew-%. Daraus werden mengenmässig je nach Anwendungsbedarf klare Zwischenverdünnungen hergestellt, in denen durch Zugabe von Trinkwasser im Gewichtsverhältnis 5:1 bis 50:1 die Wirkstoffkonzentration entsprechend herabgesetzt ist, ohne dass die Stabilität der Zwischenverdünnungen darunter leidet. Die über einen Zeitraum von mehreren Tagen stabilen Wirkstoffzwischenverdünnungen werden in den Verteilungsvorrichtungen (Dispensern) am Einsatzort unter normalen Temperaturbedingungen aufbewahrt. Die für die Behandlung der Tiere erforderlichen Mengen an Wirkstofflösung werden dann diesen Dispensern automatisch entnommen, wobei durch entsprechende Dosierungsvorrichtungen unter Zugabe von Trinkwasser die für die Therapie jeweils benötigte Endkonzentration präzise eingestellt wird. Bei dieser Endverdünnung liegt der Wirkstoffanteil im allgemeinen unter 1 Gew.-%, teilweise sogar unter 0,1 Gew.-% bis zu 100 ppm. Die Stabilitätseigenschaften der erfindungsgemässen Formulierungen, die auch bei starken Verdünnungen mit Wasser vorliegen, gestatten durch Anwendung des beschriebenen Verdünnungsprinzips eine exakte Festlegung der zur Applikation gelangenden Wirkstoffdosis, womit eine genaue Kontrolle und Steuerung der jeweiligen Therapie gegeben ist.

Gegen die zur Herstellung der erfindungsgemässen Formulierungen verwendeten funktionellen Hilfsstoffe wie Lösungsmittel und Lösungsvermittler sind hinsichtlich ihrer toxikologischen Unbedenklichkeit bei oraler Verabreichung in der erfindungsgemäss beschriebenen Weise in der Fachwelt bisher keine Einwände bekannt geworden.

Die in den erfindungsgemässen Formulierungen enthaltenen Wirkstoffkombinationen besitzen ein breites antibakterielles und antiprotozoäres Wirkungsspektrum, unter anderem vor allem gegen Escherichia coli, Kokzidien, Salmonellen, Staphylokokken und Streptokokken und sind deshalb zur prophylaktischen und therapeutischen Behandlung von Infektionskrankheiten bei Haus- und Nutztieren wie Geflügel, Schweinen, Kälbern, Rindern, Schafen und Ziegen besonders geeignet. Dabei gewinnen die erfindungsgemässen Formulierungen als Wirkstoffträger für die Trinkwassermedikation dank der Hervorbringung von hervorragenden Löslichkeitseigenschaften gerade für die Behandlung von grösseren Tierbeständen, wie sie heutzutage besonders in der landwirtschaftlichen Intensivproduktion üblich sind, eine wichtige Bedeutung.

Die in den erfindungsgemässen Formulierungen verwendeten Wirkstoffe sind bekannt. So ist das 2-Diamino-5-(3′,4′,5′-trimethoxybenzyl)-pyrimidin (Trimethoprim) in Brit. Pharmakopoeia 1973, 484 und das N-[6-Chlorpyridazinyl-(3)]-sulfanilamid (Sulfachlorpyridazin) in Handbook of Veterinary Drugs, Ed. Springer New York 1974, 564 sowie in Ullmanns Enzyklopädie d. Techn. Chemie, Verlag Chemie Weinheim, 3. Aufl. Bd. 15 und das N-[6-Chlorpyrazinyl-(2)]-sulfanilamid (Sulfachlorpyrazin) in Handbook of Veterinary Drugs, Ed. Springs New York 1974, 564 beschrieben.

Ferner sind die als funktionelle Hilfsstoffe verwendeten Lösungsmittel, Lösungsvermittler und Tenside der Fachwelt als solche bekannt.

Die erfindungsgemässen Wirkstofformulierungen werden nach folgendem Verfahren hergestellt, indem man die Wirkstoffe zusammen mit einem Lösungsvermittler, einem Tensid in einem geeigneten abdunkelbaren Gefäss, möglichst unter Ausschluss von Licht unter ständigem Rühren im grössten Teil des vorgesehenen Lösungsmittels und Wasser bei 10 bis 70 °C, vorzugsweise 10 bis 50 °C, löst, anschliessend mit dem Rest des Lösungsmittels auffüllt und falls nötig unter Verwendung von Filtrierhilfsmitteln, beispielsweise Kieselgur, filtriert. Zur Beschleunigung der Filtration kann die Temperatur gegenüber dem Lösungsvorgang leicht erhöht werden. Ferner ist es möglich, den erfindungsgemässen Formulierungen zusätzlich Konservierungsstoffe, wie beispielsweise Sorbinsäure, zuzufügen.

Die folgenden Beispiele, die die erfindungsgemässen Wirkstofformulierungen veranschaulichen, wurden bei Raumtemperatur durchgeführt, wobei unter Raumtemperatur gemäss Ph. Eur. Bd I der Temperaturbereich von 15 bis 25 °C zu verstehen ist.

Beispiel 1

15,48 g Sulfachlorpyridazin, 3,33 g Trimethoprim, 3,0 g Hydroxyäthyltheophyllin, 0,5 g Bis-(2-äthylhexyl)-Na-sulfosuccinat werden in 8,0 g entmineralisiertem Wasser und 70,0 g N-Methylpyrrolidon sowie 4,0 g Äthanolamin in einem geeigneten, abdunkelbaren Gefäss unter häufigem Umschütteln gelöst, weitere 6,88 g N-Methylpyrrolidon dazugegeben und nötigenfalls unter Zusatz eines Filterhilfsmittels filtriert.

Beispiel 2

16,67 g Sulfachlorpyridazin-Natrium, 3,33 g Trimethoprim, 3,0 g Nicotinsäureamid und 0,5 g Bis-(2-äthylhexyl)-Na-sulfosuccinat werden in 20,0 g Wasser und 60,0 g N-Methylpyrrolidon in einem geeigneten, abdunkelbaren Gefäss unter wiederholtem Umschütteln gelöst, weitere 8,21 g N-Methylpyrrolidon dazugegeben und nötigenfalls unter Verwendung eines Filterhilfsmittels filtriert.

Beispiel 3

14,72 g Sulfachlorpyrazin, 3,33 g Trimethoprim, 5,0 g Hydroxyäthyltheophyllin, 0,5 g Bis-(2-äthylhexyl)-Na-sulfosuccinat werden in 9,0 g Wasser und 70,0 g N-Methylpyrrolidon sowie 4,0 g Äthanolamin in einem geeigneten, abdunkelbaren Gefäss unter wiederholtem Umschütteln gelöst, weitere 4,83 g N-Methylpyrrolidon dazugegeben und nötigenfalls unter Verwendung eines Filterhilfsmittels filtriert.

Beispiel 4

15,48 g Sulfachlorpyridazin, 3,33 g Trimethoprim, 3,0 g Hydroxyäthyltheophyllin, 0,5 g Bis-(2-äthylhexyl)-Na-sulfosuccinat und 0,2 g Sorbinsäure werden in 8,0 g entmineralisiertem Wasser, 4,0 g Äthanolamin und 60,0 g N-Methylpyrrolidon in einem geeigneten, abdunkelbaren Gefäss unter wiederholtem Umschütteln gelöst, weitere 6,81 g N-Methylpyrrolidon dazugegeben und nötigenfalls unter Verwendung eines Filterhilfsmittels filtriert.

Beispiel 5

16,67 g Sulfachlorpyridazin-Natrium, 3,33 g Trimethoprim, 3,0 g Natrium-benzoat und 0,5 g Bis-(2-äthylhexyl)-Na-sulfosuccinat werden in einem geeigneten, abdunkelbaren Gefäss unter wiederholtem Schütteln in 20,0 g entmineralisiertem Wasser und 60,0 g N-Methylpyrrolidon gelöst, weitere 8,79 g N-Methylpyrrolidon dazugegeben und, falls nötig, unter Verwendung eines Filterhilfsmittels, filtriert.

Beispiel 6

16,67 g Sulfachlorpyrazin-Natrium-Monohydrat, 3,33 g Trimethoprim, 3,0 g Hydroxyäthyltheophyllin und 0,5 Bis-(2-äthylhexyl)-Na-sulfosuccinat werden in einem geeigneten, abdunkelbaren Gefäss unter wiederholtem Umschütteln in 8,0 g Wasser, 19,6 g Glykolmonoäthyläther und 50,0 g N-Methylpyrrolidon gelöst, weitere 7,79 g N-Methylpyrrolidon dazugegeben und nötigenfalls unter Verwendung eines Filterhilfsmittels filtriert.

**Patentansprüche**

1. Stabile flüssige Formulierung, enthaltend 2,6-Diamino-5-(3′,4′,5′-trimethoxybenzyl)-pyrimidin (Trimethoprim)
und ein Sulfonamid der Formel I

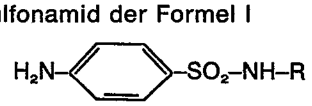 (I)

in welcher

R 6-Chlor-2-pyrazinyl oder 6-Chlor-3-pyridazinyl bedeutet, oder ein Salz davon im Gewichtsverhältnis 1:1 bis 1:20 mit einem Gesamtanteil von 15–25 Gew.-% bezogen auf das Gesamtgewicht der Formulierung, ein physiologisch verträgliches mit Wasser mischbares Lösungsmittel, einen Lösungsvermittler, ein Tensid und Wasser und für den Fall, dass das Sulfonamid nicht als Salz eingesetzt wird, eine Base, dadurch gekennzeichnet, dass als Lösungsmittel N-Methylpyrrolidon allein oder in Kombination mit Glykolmonoäthyläther neben Wasser, als Tensid Bis-(2-äthylhexyl)-Na-sulfosuccinat und als Lösungsvermittler einen aus der Gruppe Hydroxyäthyltheophyllin, Nicotinsäureamid oder Na-Benzoat und als Base Äthanolamin verwendet werden, wobei die verwendete Menge des Lösungsmittels 60–80 Gew.-%, des Tensids 0,1–1,0 Gew.-% und des Lösungsvermittlers 1–10 Gew.-% bezogen auf das Gesamtgewicht der Formulierung beträgt.

2. Stabile flüssige Formulierung gemäss Anspruch 1, dadurch gekennzeichnet, dass das Gewichtsverhältnis von Trimethoprim zum Sulfonamid der Formel I oder einem seiner Salze 1:4 bis 1:5 beträgt.

3. Stabile flüssige Formulierung gemäss den Ansprüchen 1 und 2, dadurch gekennzeichnet, dass als Lösungsvermittler 1–10 Gew.-% Hydroxyäthyltheophyllin oder Na-Benzoat bezogen auf das Gesamtgewicht der Formulierung verwendet werden.

4. Stabile flüssige Formulierung gemäss den Ansprüchen 1 und 2, dadurch gekennzeichnet, dass als Lösungsvermittler 2–4 Gew.-% Nicotinsäure bezogen auf das Gesamtgewicht der Formulierung verwendet wird.

5. Verfahren zur Herstellung einer Wirkstoffkombination gemäss den Ansprüchen 1–4, dadurch gekennzeichnet, dass man die Wirkstoffe zusammen mit einem Lösungsvermittler aus der Gruppe Hydroxyäthyltheophyllin, Nicotinsäureamid oder Na-Benzoat, dem Tensid Bis-(2-äthylhexyl)-Na-sulfosuccinat im grössten Teil des Lösungsmittels N-Methylpyrrolidon allein oder in Kombination mit Glykolmonoäthyläther und Wasser bei 10 bis 70 °C löst und anschliessend die gebildete Lösung mit dem Rest des Lösungsmittels auffüllt und gegebenenfalls unter Anwendung von Filterhilfsmitteln und Temperaturerhöhung filtriert.

6. Verfahren zur Herstellung einer Wirkstoffkombination gemäss Anspruch 5, dadurch gekennzeichnet, dass das Verfahren bei 10 bis 50 °C durchgeführt wird.

7. Verwendung der Formulierung gemäss der Ansprüche 1–4, dadurch gekennzeichnet, dass diese in durch Zugabe von Trinkwasser im Gewichtsverhältnis von 5:1 bis 50:1 zwischenverdünnter Form mit automatischen Dosiergeräten den zur Verabreichung an Tiere vorgesehenen üblichen Tränken laufend beigemischt wird.

**Claims**

1. A stable liquid formulation containing 2,6-diamino-5-(3′,4′,5′-trimethoxybenzyl)-pyrimidine (trimethoprim)

and a sulfonamide of the formula I

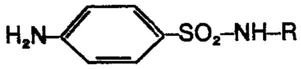 (I)

wherein
R is 6-chloro-2-pyrazinyl or 6-chloro-3-pyridazinyl or a salt thereof, in the quantity ration of 1:3 to 1:12, with a total proportion of 15–25 per cent by weight, relative to the total weight of the formulation, a physiologically compatible water-miscible solvent, a solubilizer, a surfactant and water, and a base in the case where the sulfonamide is not used as a salt, in which composition the solvent used is N-methylpyrrolidone, on its own or in combination with glycol monoethyl ether, in addition to water, the surfactant used is bis-(2-ethylhexyl)-Na-sulfosuccinate, and the solubilizer used is one from the group comprising hydroxyethyltheophylline, nicotinic acid amide and sodium benzoate, and the base used is ethanolamine, the employed amount of solvent being 60–80 per cent by weight, of tenside 0.1–1.0 per cent by weight and of solubilizer 1–10 per cent by weight, relative to the total weight of the formulation.

2. A stable liquid formulation according to Claim 1, wherein the weight ratio of trimethoprim to sulfonamide of the formula I or to a salt thereof is 1:4 to 1:5.

3. A stable liquid formulation according to Claims 1 and 2, wherein there is used as solubilizer 1–10 per cent by weight of hydroxyethyltheophylline or of sodium benzoate, relative to the total weight of the formulation.

4. A stable liquid formulation according to Claims 1 and 2, wherein there is used as solubilizer 2–4 per cent by weight of nicotinic acid, relative to the total weight of the formulation.

5. A process for producing an active-substance combination according to Claims 1–4, wherein the active substances, together with a solubilizer, chosen from the group comprising hydroxyethyltheophylline, nicotinic acid amide and sodium benzoate, and the surfactant bis-(2-ethylhexyl)-Na-sulfosuccinate, are dissolved in the major part of the solvent N-methylpyrrolidone, on its own or in combination with glycol monoethyl ether, and water at 10 to 70 °C; the resulting solution is subsequently made up to the required amount with the remainder of the solvent, and then filtered, if necessary using filtering auxiliaries and raising the temperature of the solution.

6. A process for producing an active-substance combination according to Claim 5, wherein the process is performed at 10 to 50 °C.

7. Use of the formulation according to Claims 1–4, which comprises continuously mixing this formulation, in the form it is in after it has been intermediately diluted by the addition of drinking water in the weight ratio of 5:1 to 50:1, by means of automatic dosing devices, with the customary drinking water provided for the animals.

**Revendications**

1. Formulation liquide stable contenant de la 2,6-diamino-5-(3′,4′,5′-triméthoxybenzyl)-pyrimidine (triméthoprim)
et un sulfonamide de formule I

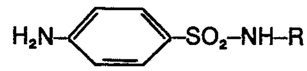 (I)

où R représente un 6-chloro-2-pyrazinyle ou un 6-chloro-3-pyrazinyle, ou un sel de ce corps dans un rapport pondéral de 1:3 à 1:12 avec une fraction totale de 15 à 25% en poids par rapport au poids total de la formulation, un solvant miscible à l'eau physiologiquement acceptable, un tiers solvant, un agent tensioactif et de l'eau, et pour les cas où le sulfonamide n'est pas utilisé sous forme de sel, une base, caractérisée en ce qu'on utilise comme solvant la N-méthylpyrrolidone seule ou en combinaison avec du monoéthyléther de glycol outre l'eau, comme agent tensioactif le sulfosuccinate de bis-(2-éthylhexyl)-sodium et comme tiers solvant un corps du groupe constitué par l'hydroxyéthylthéophylline, l'amide de l'acide nicotinique ou le benzoate de sodium et comme base l'éthanolamine, la quantité de solvant utilisée s'élevant à 60–80% en poids, celle de l'agent tensioactif à 0,1–1,0% en poids, et celle de tiers solvants à 1–10% en poids par rapport au poids total de la formulation.

2. Formulation liquide stable selon la revendication 1, caractérisée en ce que le rapport pondéral du triméthoprim au sulfonamide de formule I ou à l'un de ces sels s'éléve à 1:4 à 1:5.

3. Formulation liquide stable selon les revendications 1 et 2 caractérisée en ce qu'on utilise comme tiers solvant de 1 à 10% en poids d'hydroxyéthylthéophylline ou de benzoate de sodium par rapport au poids total de la formulation.

4. Formulation liquide stable selon les revendications 1 et 2, caractérisée en ce qu'on utilise comme tiers solvant de 2 à 4% en poids d'acide nicotinique par rapport au poids total de la formulation.

5. Procédé de préparation d'une combinaison de substance active selon les revendications 1–4, caractérisé en ce qu'on dissout les substances actives avec un tiers solvant du groupe de l'hydroxyéthylthéophylline, de l'amide de l'acide nicotinique ou du benzoate de sodium, avec l'agent tensioactif sulfosuccinate de bis-(2-éthylhexyl)-sodium dans la plus grande partie du solvant N-méthylpyrrolidone seul ou en combinaison avec du monoéthyléther de glycol et de l'eau entre 10 et 70 °C puis en ce qu'on complète la solution formée avec le reste du solvant et éventuellement en ce qu'on filtre en utilisant des auxiliaires de filtration et une élévation de température.

6. Procédé de préparation d'une combinaison de substance active selon la revendication 5, caractérisé en ce qu'on conduit le procédé entre 10 et 50 °C.

7. Application de la formulation selon les revendications 1–4, caractérisée en ce qu'on les mélange de façon continue sous forme diluée de façon intermédiaire par addition d'eau de boisson dans un rapport pondéral de 5:1 à 50:1 avec des appareils de dosage automatiques aux boissons habituelles prévues pour l'administration aux animaux.